# EUROPÄISCHE PATENTSCHRIFT

(11) **EP 1 670 746 B1**
(45) Veröffentlichungstag und Bekanntmachung des Hinweises auf die Patenterteilung: **18.01.2012**
(21) Anmeldenummer: 04787012.6
(22) Anmeldetag: 24.09.2004
(51) Int. Cl.: C07C 209/16, C07C 211/04

(54) **VERFAHREN ZUR REGELUNG DER REAKTOREINTRITTSTEMPERATUR BEI DER METHYLAMINHERSTELLUNG**
METHOD FOR CONTROLLING THE REACTOR ADMISSION TEMPERATURE DURING THE PRODUCTION OF METHYLAMINE
PROCEDE POUR REGULER LA TEMPERATURE D'ENTREE DE REACTEUR DANS LE CADRE DE LA PREPARATION DE METHYLAMINES

(30) Priorität: 24.09.2003 DE 10344283
(43) Veröffentlichungstag der Anmeldung: 21.06.2006
(73) Patentinhaber: BASF SE, 67056 Ludwigshafen (DE)
(72) Erfinder: REUTEMANN, Werner, 67240 Bobenheim-Roxheim (DE); WAMBSGANSS , Rolf, 76829 Landau (DE); POPLOW, Frank, 67065 Ludwigshafen (DE); WEBER, Theodor, 67063 Ludwigshafen (DE); ROSS , Karl-Heinz, 67269 Grünstadt (DE); JULIUS, Manfred, 67117 Limburgerhof (DE)
(86) Internationale Anmeldenummer: PCT/EP2004/010765
(87) Internationale Veröffentlichungsnummer: WO 2005/028416

(56) Entgegenhaltungen:
- DATABASE WPI Section Ch, Week 198516 Derwent Publications Ltd., London, GB; Class E16, AN 1985-096654 XP002323350 & JP 60 045550 A (NITTO CHEM IND CO LTD) 12. März 1985 (1985-03-12) in der Anmeldung erwähnt

## Beschreibung

Die Erfindung betrifft ein Verfahren zur Regelung der Reaktoreintrittstemperatur bei der Herstellung von Methylaminen durch Gasphasenreaktion aus Methanol und Ammoniak in Gegenwart eines heterogenen Katalysators. Damit die Reaktion zur Herstellung der Methylamine abläuft, müssen die Edukte bereits vorgeheizt dem Reaktor zugeführt werden.

In dem bei der Umsetzung von Methanol und Ammoniak zu Methylaminen anfallenden Produktstrom sind neben Monomethylamin, Dimethylamin und Trimethylamin Wasser sowie nicht umgesetzter Ammoniak und nicht umgesetztes Methanol enthalten. Die Reaktion von Methanol und Ammoniak zu Methylamin ist exotherm und erfolgt in Gegenwart eines heterogenen Katalysators. Zur Durchführung der Reaktion werden die Edukte Methanol und Ammoniak dem Reaktor vorgeheizt zugeführt.

Bei der Reaktion von Methanol und Ammoniak zu Methylamin entsteht mehr Trimethylamin als benötigt wird. Das überschüssige Trimethylamin wird dem Reaktorzulauf zugegeben und im Reaktor in einer endothermen Reaktion zu Dimethylamin und Monomethylamin umgesetzt. Die Vorheizung der zugeführten Edukte erfolgt in dem Reaktor vorgeschalteten Wärmetauschern. Zur Vorheizung kann wie in JP-A2-60 045 550 beschrieben der bei der Reaktion anfallende Produktgasstrom eingesetzt werden. Hierzu wird der Produktgasstrom durch die zur Vorheizung eingesetzten Wärmetauscher geleitet. Aufgrund der in Summe exothermen Reaktionen nimmt die Temperatur im Reaktor zwischen Reaktoreinlauf und Reaktorablauf zu. Aufgrund der hohen Temperatur am Reaktorablauf wird bei Verwendung des Produktgasstromes zur Aufheizung der Edukte im stationären Betrieb keine weitere Energie zur Vorheizung der Edukte benötigt.

Die erforderlichen Temperaturen im Reaktor sind durch technische Begrenzungen festgelegt. Die Reaktoreintrittstemperatur muss so hoch sein, dass die Reaktion im Reaktor sofort anspringt. Weiterhin darf die Reaktorhöchsttemperatur nicht zu hoch sein, da ansonsten vermehrt eine Zersetzung der Methylamine zu gasförmigen und festen Abbauprodukten stattfindet. Die Reaktorhöchsttemperatur muss jedoch so hoch gewählt sein, dass das Methanol weitgehend umgesetzt wird und das thermische Gleichgewicht erreicht wird.

Bei den derzeit eingesetzten Verfahren zur Vorheizung der Edukte kann es aufgrund von Temperaturschwankungen in der Umgebung oder durch unterschiedliche Mengen an zu recyclierendem Trimethylamin zu Schwankungen bei der Reaktoreintrittstemperatur kommen. Die Schwankungen der Reaktoreintrittstemperatur führen auch zu Schwankungen bei der Reaktorhöchsttemperatur. Im ungünstigsten Fall wird die Reaktorhöchsttemperatur so hoch, dass sich die Methylamine wieder zersetzen.

Aufgabe der Erfindung war es daher, ein Verfahren zur Herstellung von Methylaminen aus Methanol und Ammoniak durch Gasphasenreaktion bereitzustellen, bei dem die Reaktoreintrittstemperatur und/oder Austrittstemperatur innerhalb eines engen Bereiches geregelt wird. Weiterhin ist das Verfahren zur Herstellung von Methylamin so zu gestalten, dass zur Vorheizung und Überhitzung der Edukte im stationären Betrieb möglichst keine Energie von außen zugeführt werden muss.

Die Lösung zur Regelung der Reaktoreintrittstemperatur und/oder Austrittstemperatur bei dem Verfahren zur Herstellung von Methylamin besteht darin, den Eduktgasstrom vor dem Eintritt in den Reaktor oder den Produktgasstrom nach dem Verlassen des Reaktors durch ein verstellbares Ventil zu leiten.

Zur Herstellung von Methylamin aus Methanol und Ammoniak als Edukten werden zunächst die Edukte in einem oder mehreren Wärmetauschern verdampft und überhitzt. Die so auf eine Temperatur im Bereich von 350°C bis 450°C, bevorzugt im Bereich von 350°C bis 400°C und besonders bevorzugt im Bereich von 360°C bis 370°C erhitzten Edukte werden mit dieser Temperatur als Reaktoreintrittstemperatur einem Reaktor zugeführt.

Neben Methanol und Ammoniak werden dem Reaktor auch die bei der Reaktion entstehenden Reaktionsnebenprodukte zugeführt. Die Reaktionsprodukte werden dazu in einer Nachbehandlung nach der Reaktion aus dem Produktgasstrom abgetrennt.

Im Reaktor werden die Edukte in Gegenwart eines heterogenen Katalysators bei einem Druck im Bereich von 15 bis 30 bar zu Monomethylamin, Dimethylamin und Trimethylamin umgesetzt. Die Reaktion zur Herstellung der Methylamine aus Methanol und Ammoniak ist exotherm, daher nimmt die Temperatur im Reaktor zu. Aus dem Reaktor wird ein Produktgasstrom, der neben Monomethylamin, Dimethylamin und Trimethylamin nicht umgesetztes Methanol und nicht umgesetzten Ammoniak sowie bei der Reaktion als Nebenprodukt erzeugtes Wasser und weitere Nebenprodukte wie Kohlenmonoxid und Kohlendioxid enthält abgezogen.

Aufgrund der exothermen Reaktion nimmt die Temperatur von Reaktoreintritt zu Reaktoraustritt zu. Damit die Temperatur im Reaktor nicht über 450°C steigt, kann durch Kühlung des Reaktors Reaktionswärme abgeführt werden.

Bei der Bildung der Methylamine entsteht meist mehr Trimethylamin als genutzt werden kann. Das nicht genutzte Trimethylamin wird mit dem Eduktstrom erneut der Reaktion zugeführt. Die Umsetzung von Trimethylamin zu Dimethylamin ist endotherm. Hierzu wird bei der exothermen Reaktion von Ammoniak und Methanol zu Methylaminen freiwerdende Reaktionswärme verbraucht. Aus diesem Grund kann der Reaktor auch adiabat betrieben werden.

Die zum Anfahren der Reaktion erforderliche Wärme kann durch eine zusätzliche Aufheizung der Edukte zugeführt werden.

Bei dem erfindungsgemäßen Verfahren zur Herstellung von Methylamin wird der Gasstrom nach Verdampfung und Überhitzung der Edukte durch ein Ventil geleitet. Durch das Ventil kann der Druck des Gasstromes und damit die Kondensationstemperatur nach dem Reaktor variiert werden. Das Ventil kann dazu vor oder hinter dem Reaktor eingebaut sein.

Zur Wärmeabgabe an die Edukte wird der Produktgasstrom in dem einen oder den mehreren Wärmetauschern zum Eduktstrom vorzugsweise im Gegenstrom geführt. Bei der Wärmeabgabe zum Aufheizen der Edukte wird der Produktstrom in den Wärmetauschern zumindest teilweise kondensiert. Um Erosionen durch mitgerissene Kondensattröpfchen zu vermeiden, kann den einzelnen Wärmetauschern jeweils ein Kondensatabscheider nachgeschaltet sein.

Durch eine im Ventil herbeigeführte Querschnittsverengung und die damit verbundene Druckabnahme des Gasstromes hinter dem Ventil wird die Kondensationstemperatur des Produktgasstromes abgesenkt. Aufgrund der niedrigeren Kondensationstemperatur des Produktgasstromes wird der Eduktstrom weniger stark aufgeheizt. Hierdurch nimmt die Reaktoreintrittstemperatur ab. Wenn andererseits das Ventil weiter geöffnet wird und damit der Druck hinter dem Ventil zunimmt, steigt auch die Kondensationstemperatur des Produktgasstromes. Auf diese Weise wird die Reaktoreintrittstemperatur der Edukte erhöht.

Wenn die vom Produktgasstrom übertragbare Wärme zum Vorheizen der Edukte nicht ausreicht, kann dem Produktgasstrom vorzugsweise im Zulauf zu einem der Wärmetauscher Wasserdampf zugegeben werden. Durch den zusätzlichen Wasserdampf wird die an die Edukte übertragene Wärmemenge erhöht.

Neben der Zugabe von Wasserdampf besteht auch die Möglichkeit, wenn die vom Produktgasstrom übertragene Wärmemenge zu groß ist, einen Teil des Produktgasstromes vor dem Eintritt in die Wärmetauscher abzuziehen. Dabei kann die Abzweigung eines Teilstroms des Produktgasstromes an jeder dem Fachmann bekannten Stelle zwischen den Wärmetauschern oder im Wärmetauscher erfolgen. Die Menge des abgezweigten Produktgasteilstromes wird dabei vorzugsweise durch ein Ventil gesteuert.

Eine weitere Möglichkeit zur Regelung der Temperatur entsprechend der erfindungsgemäßen Lösung zur Herstellung von Methylamin besteht darin, die Zumischstelle des Methanolstromes in den ammoniakhaltigen Eduktstrom zu variieren. Die Zumischung des Methanols kann dabei an jeder belieben, dem Fachmann bekannten Stelle zwischen den einzelnen Wärmeübertragern oder an jeglicher beliebigen dem Fachmann bekannten Stelle in einem Wärmetauscher erfolgen. Vorzugsweise erfolgt die Zugabe des Methanols dabei jedoch zwischen zwei Wärmetauschern oder hinter dem letzten Wärmetauscher. Zudem kann der Ammoniak und Reaktionsnebenprodukte enthaltende Eduktstrom vor der Zumischung des Methanols vorgeheizt werden.

Das Ventil zur Variation des Druckes des Produktgasstromes ist vorzugsweise so gestaltet, dass der Druck am Ventileinlass um 0 bis 5 bar höher liegt am Ventilauslass. Weiterhin ist das Ventil vorzugsweise stufenlos verstellbar.

Ein Ventil im Sinne der vorliegenden Erfindung ist ein Ventil, bei dem ein Absperrkörper, zum Beispiel eine Platte, ein Kegel, ein Kolben oder eine Kugel mit einer Abhebbewegung einen zylindrischen Ringquerschnitt parallel zur Strömungsrichtung freigibt, ein Schieber, bei dem der Absperrkörper mit parallel oder keilförmig gestellten Flächen einen Strömungsquerschnitt quer zur Strömungsrichtung freigibt oder ein Hahn oder einen Drehschieber, bei denen der Absperrkörper um seine Achse quer zur Strömungsrichtung gedreht und dadurch einen Strömungsquerschnitt freigibt. Neben den genannten Bauarten ist als Ventil auch jede weitere dem Fachmann bekannte Bauart zum Absperren von Rohrquerschnitten, bei denen sich der Rohrquerschnitt variieren lässt, zu verstehen.

Als Wärmetauscher zum Vorheizen der Edukte können alle dem Fachmann bekannten Wärmetauscherbauarten eingesetzt werden. Als Wärmetauscher eignen sich zum Beispiel Rohrbündelwärmetauscher, Spiralwärmetauscher oder Plattenwärmetauscher. Vorzugsweise werden jedoch Rohrbündelwärmetauscher eingesetzt. Die eingesetzten Wärmetauscher können dabei im Gleichstrom, Gegenstrom oder Kreuzstrom betrieben werden. Weiterhin ist jede dem Fachmann bekannte Kombination aus Kreuz-, Gegen- und Gleichstrom möglich. So kann zum Beispiel bei Einsatz mehrerer Wärmetauscher zumindest ein Wärmetauscher im Gleichstrom betrieben werden, während die restlichen Wärmetauscher im Gegenstrom arbeiten. Die bevorzugte Betriebsart der Wärmetauscher zur Aufheizung der Edukte ist der Gegenstrom.

Um die Reaktoreintrittstemperatur regeln zu können, werden an verschiedenen Stellen der Anlage zur Herstellung von Methylamin Messwerte erfasst. So kann zum Beispiel vor der Zumischung des Methanols in den ammoniakhaltigen Eduktstrom der Durchfluss des ammoniakhaltigen Eduktstromes und der Durchfluss des Methanolstromes gemessen werden. Weiterhin kann bei Zumischung des Methanols an verschiedenen Positionen des ammoniakhaltigen Eduktstromes der Durchfluss der einzelnen Methanolteilströme gemessen werden. Bei einer Auftrennung des Produktstromes in mehrere Teilströme kann in jedem Teilstrom eine Durchflussmessung erfolgen. Schließlich kann durch eine Durchflussmessung die Menge an zusätzlichem Dampf zur Aufheizung der Edukte gemessen werden.

Zur Regelung der Reaktoreintrittstemperatur ist es weiterhin erforderlich, dass die Reaktoreintrittstemperatur gemessen wird. Neben der Reaktoreintrittstemperatur können auch die Temperatur des ammoniakhaltigen Eduktstromes, des Methanols und die Temperatur am Reaktorausgang gemessen werden. Schließlich kann vorzugsweise noch eine Differenzdruckmessung zwischen dem Zulauf zum ersten Wärmetauscher und dem den ersten Wärmetauscher verlassenden zur Vorheizung der Edukte eingesetzten Produktstrom erfolgen.

Die in den Kondensatabscheidern anfallenden flüssigen Produktströme und der den ersten Wärmetauscher verlassende Produktstrom werden vorzugsweise einer Weiterbehandlung zur Abtrennung der Methylamine zugeführt.

Im Folgenden wird die Erfindung anhand einer Zeichnung näher beschrieben.

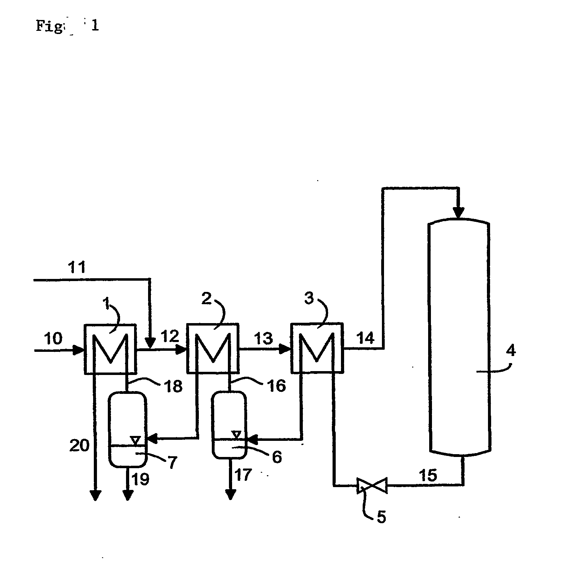

Figur 1 zeigt ein Fließbild eines erfindungsgemäß ausgebildeten Verfahrens zur Herstellung von Methylaminen.

Bei dem in Figur 1 dargestellten Verfahren zur Herstellung von Methylamin bildet ein ammoniakhaltiger Eduktstrom einen Zulauf 10 zu einem ersten Vorwärmer 1. Der Zulauf 10 kann neben Ammoniak auch bei der Methylaminherstellung erhaltene Nebenprodukte sowie überschüssige Methylamine enthalten. Bei dem in Figur 1 dargstellten Verfahren wird der Zulauf 10 im ersten Vorwärmer 1 erwärmt, anschließend mit einem Methanolzulauf 11 vermischt und als Zulauf 12 einem zweiten Vorwärmer 2 zugeführt. Im Weiteren werden die im Zulauf 12 in dem zweiten Vorwärmer 2 enthaltenen Stoffe als Edukte bezeichnet. In Figur 1 werden die im zweiten Vorwärmer 2 weitererwärmten Edukte über einen Zulauf 13 einem Überhitzer 3 zugeführt. Im Überhitzer 3 werden die Edukte auf die Reaktoreintrittstemperatur überhitzt. Die so auf Reaktoreintrittstemperatur erhitzten Edukte werden über einen Reaktorzulauf 14 einem Reaktor 4 zugeführt. Im Reaktor 4 erfolgt die exotherme Umsetzung von Methanol und Ammoniak zu Monomethylamin, Dimethylamin und Trimethylamin. Zudem wird das in großen Mengen bei Reaktion entstehende, aber nur in geringen Mengen benötigte Trimethylamin, welches dem Reaktor über den Eduktstrom wieder zugeführt wurde, in einer endothermen Reaktion zu Dimethylamin und Monomethylamin umgesetzt. In der Summe sind die im Reaktor 4 ablaufenden Reaktionen jedoch exotherm. Aus diesem Grund nimmt die Temperatur entlang des Reaktors 4 zu. Über einen Reaktoraustrag 15 werden die Reaktionsprodukte abgezogen. Der über den Reaktoraustrag 15 abgezogene Produktgasstrom wird über ein Ventil 5 geleitet, mit dem der Druck vor dem Ventil 5 variiert werden kann. Neben der in Figur 1 dargestellten Ausführungsform, bei der das Ventil 5 hinter dem Reaktor 4 angeordnet ist, kann das Ventil 5 auch vor dem Reaktor 4 angeordnet sein. Eine Absenkung des Druckes führt dazu, dass die Verdampfungstemperatur des Eduktgasstromes absinkt, während eine Erhöhung des Druckes dazu führt, dass die Verdampfungstemperatur des Eduktgasstromes ansteigt.

Gemäß der in Figur 1 dargestellten Ausführungsvariante wird der Produktgasstrom im Gegenstrom durch den Überhitzer 3 und den zweiten Vorwärmer 2 sowie den ersten Vorwärmer 1 geführt. Zur Vermeidung von Erosionen im ersten Vorwärmer 1, zweiten Vorwärmer 2 und Überhitzer 3 sind zwischen dem Überhitzer 3 und dem zweiten Vorwärmer 2 ein erster Tropfenabscheider 6 und dem zweiten Vorwärmer 2 und dem ersten Vorwärmer 1 ein zweiter Tropfenabscheider 7 zwischengeschaltet. In den Tropfenabscheidern 6, 7 wird das im Überhitzer 3 und dem zweiten Vorwärmer 2 angefallene Kondensat aus dem Produktgasstrom abgetrennt. Das im ersten Tropfenabscheider 6 angefallene Kondensat wird über einen Kondensatablauf 17 einer in Figur 1 nicht dargestellten Produktaufbereitung zugeführt. Analog wird auch aus dem zweiten Tropfenabscheider 7 das anfallende Kondensat über einen Kondensatablauf 19 der Produktaufbereitung zugeführt. Der den ersten Vorwärmer 1 verlassende Produktstrom wird über einen Produktaustrag 20 ebenfalls der Produktaufbereitung zugeführt. In der Produktaufbereitung werden aus dem Produktstrom Monomethylamin, Dimethylamin und Trimethylamin abgetrennt. Da bei der Reaktion zu Dimethylamin weit mehr Trimethylamin anfällt als kommerziell benötigt wird, wird das überschüssige Trimethylamin über den Zulauf 10 erneut der Reaktion zugeführt. Entsprechend wird auch überschüssiges Monomethylamin erneut über den Zulauf 10 der Reaktion zugeführt.

Neben der Einstellung der Reaktorzulauftemperatur durch Einstellung des Druckes über das Ventil 5 besteht auch die Möglichkeit, die Reaktoreintrittstemperatur dadurch zu regeln, dass in den Produktgasstrom nach dem Ventil 5, in den Heizmediumszulauf 16, 18 in den zweiten Vorwärmer 2 oder in den Heizmediumszulauf 18 in den ersten Vorwärmer 1 Wasserdampf zugegeben wird. Neben der Zugabe des Dampfes in den Heizmediumszulauf 16, 18 eines der Wärmetauscher 1, 2, 3 kann der Dampf auch direkt in das Heizmedium in einem der Wärmetauscher 1, 2, 3 zugegeben werden. Neben dem Methanolzulauf 11 zwischen dem ersten Vorwärmer 1 und dem zweiten Vorwärmer 2 kann das Methanol auch in den Zulauf 10 zum ersten Vorwärmer 1 oder den Zulauf 13 in den Überhitzer 3 oder direkt in den Reaktorzulauf 14 zugeführt werden. Neben der Zugabe des gesamten Methanols in den Zulauf 10 zum ersten Vorwärmer 1, den Zulauf 12 in den zweiten Vorwärmer 2, den Zulauf 13 in den Überhitzer 3 oder den Reaktorzulauf 14 kann der Methanolzulauf 11 auch in einzelne Teilströme aufgeteilt werden. Dabei können die Teilströme an unterschiedlicher Position dem Eduktstrom zugemischt werden.

Neben der in Figur 1 dargestellten Ausführungsvariante mit einem ersten Vorwärmer 1, einem zweiten Vorwärmer 2 und einem Überhitzer 3 ist es auch möglich, nur einen Wärmetauscher zum Verdampfen und Überhitzen einzusetzen, einen Wärmetauscher zum Verdampfen und einen Wärmetauscher zum Überhitzen. Auch ist jede beliebige andere Anzahl an Wärmetauschern zum Verdampfen und Überhitzen des Eduktstromes denkbar.

Außer dem in Figur 1 dargestellten Betrieb der Wärmetauscher 1, 2, 3 im Gegenstrom ist auch ein Betrieb im Gleichstrom oder im Kreuzstrom denkbar, sowie jede bekannte Kombination aus Gegenstrom, Gleichstrom oder Kreuzstrom.

Insbesondere bei Verwendung nur eines Wärmetauschers zum Verdampfen und Überhitzen des Eduktstromes ist vorzusehen, den Methanolzulauf 11 an jeder beliebigen Position des Wärmetauschers zu ermöglichen.

Durch die Regelung des Druckes des Produktgasstromes durch das Ventil 5, die Position des Methanolzulaufs 11 und die optionale Zugabe von Wasserdampf in den Produktstrom zur Aufheizung der Edukte lässt sich die Reaktoreintrittstemperatur auf eine Temperatur im Bereich von 360°C bis 370°C regeln. Durch die Regelung der Reaktoreintrittstemperatur auf eine Temperatur im Bereich von 360°C bis 370°C wird gewährleistet, dass die Temperatur im Reaktor 450°C nicht übersteigt. Die Temperaturerhöhung im Reaktor 4 erfolgt durch die freiwerdende Wärme bei der exothermen Reaktion zur Bildung von Methylamin. Ein Teil der Wärme wird jedoch zur endothermen Umsetzung von Trimethylamin benötigt. Um die Reaktortemperatur im Bereich von 360°C bis 450°C zu halten, kann der Reaktor adiabat betrieben werden oder es kann durch Kühlung des Reaktors 4 Reaktionswärme abgeführt werden.

Die zur Reaktion erforderliche Aktivierungsenergie wird zum Start der Reaktion vorzugsweise durch eine elektrische Aufheizung zugeführt.

### Bezugszeichenliste

- 1: erster Vorwärmer
- 2: zweiter Vorwärmer
- 3: Überhitzer
- 4: Reaktor
- 5: Ventil
- 6: erster Tropfenabscheider
- 7: zweiter Tropfenabscheider
- 10: Zulauf
- 11: Methanolzulauf
- 12: Zulauf in den zweiten Vorwärmer 2
- 13: Zulauf in den Überhitzer 3
- 14: Reaktorzulauf
- 15: Reaktoraustrag
- 16: Heizmediumszulauf in den zweiten Vorwärmer 2
- 17: Kondensatablauf
- 18: Heizmediumszulauf in den ersten Vorwärmer 1
- 19: Kondensatablauf
- 20: Produktaustrag

## Patentansprüche

1. Verfahren zur Herstellung von Metylaminen durch Gasphasenreaktion aus Methanol und Ammoniak als Edukte in Gegenwart eines heterogenen Katalysators bei einem Druck im Bereich von 15 bis 30 bar, bei dem die Edukte in einem oder mehreren Wärmetauschern (1, 2, 3) verdampft, zu einem Eduktgasstrom überhitzt und anschließend einem Reaktor (4) zugeführt werden, wobei die Edukte entweder im Zulauf zu einem der Wärmetauscher (1, 2, 3) oder an einer beliebigen anderen Position des Wärmetauschers (1, 2, 3) vermischt werden, aus dem Reaktor (4) ein Monomethylamin, Dimethylamin und Trimethylamin sowie Reaktionsnebenprodukte enthaltender Produktgasstrom abgezogen wird, **dadurch gekennzeichnet, dass** zur Regelung der Reaktoreintrittstemperatur der Edukte auf eine Temperatur im Bereich von 360°C bis 370°C der Eduktgasstrom oder Produktgasstrom zur Variation des Druckes teilweise oder vollständig durch ein verstellbares Ventil (5) geleitet wird, das vor oder hinter dem Reaktor (4) eingebaut ist.

2. Verfahren gemäß Anspruch 1, **dadurch gekennzeichnet, dass** das Ventil (5) stufenlos verstellbar ist.

3. Verfahren gemäß Anspruch 1 oder 2, **dadurch gekennzeichnet, dass** der Produktgasstrom zur Verdampfung und Überhitzung der Edukte eingesetzt wird, wobei der Produktgasstrom teilweise kondensiert.

4. Verfahren gemäß Anspruch 1 oder 2, **dadurch gekennzeichnet, dass** ein Teilstrom des Produktgasstromes zur Verdampfung und Überhitzung der Edukte verwendet wird.

5. Verfahren gemäß einem der Ansprüche 1 bis 4, **dadurch gekennzeichnet, dass** die bei der Reaktion entstehenden Reaktionsnebenprodukte aus dem Produktgasstrom abgetrennt und erneut dem Reaktor (4) zugeführt werden.

6. Verfahren gemäß Anspruch 5, **dadurch gekennzeichnet, dass** der Ammoniak und die erneut dem Reaktor (4) zugeführten Reaktionsnebenprodukte vorgeheizt werden, bevor das Methanol zugegeben wird.

7. Verfahren gemäß einem oder mehrerer der Ansprüche 1 bis 6, **dadurch gekennzeichnet, dass** zur Vorwärmung und Überhitzung der Edukte dem Produktgasstrom Wasserdampf zugefügt wird.

8. Verfahren gemäß einem oder mehrerer der Ansprüche 1 bis 7, **dadurch gekennzeichnet, dass** der Reaktor (4) adiabat betrieben wird.

9. Verfahren gemäß einem oder mehrerer der Ansprüche 1 bis 7, **dadurch gekennzeichnet, dass** durch Kühlung des Reaktors (4) Reaktionswärme abgeführt wird.

10. Verfahren gemäß einem oder mehrerer der Ansprüche 1 bis 9, **dadurch gekennzeichnet, dass** beim Einsatz mehrerer Wärmetauscher (1, 2, 3) zur Verdampfung und Überhitzung der Edukte hinter jedem Wärmetauscher (1, 2, 3) ein Tropfenabscheider (6, 7) zur Kondensatabscheidung aus dem Produktgasstrom eingesetzt wird.

11. Verfahren gemäß einem oder mehrerer der Ansprüche 1 bis 10, **dadurch gekennzeichnet, dass** der Druck am Ventileinlass um 0 bis 5 bar höher liegt als am Ventilauslass.

12. Verfahren gemäß einem oder mehrerer der Ansprüche 1 bis 11, **dadurch gekennzeichnet, dass** zum Anfahren der Reaktion die Edukte elektrisch aufgeheizt werden.

13. Verfahren gemäß einem oder mehrerer der Ansprüche 1 bis 12, **dadurch gekennzeichnet, dass** die Verdampfung und Überhitzung der Edukte im Gegenstrom erfolgt.

14. Verfahren gemäß einem oder mehrerer der Ansprüche 1 bis 12, **dadurch gekennzeichnet, dass** die Verdampfung und Überhitzung der Edukte im Gleichstrom erfolgt.

15. Verfahren gemäß einem oder mehrerer der Ansprüche 1 bis 12, **dadurch gekennzeichnet, dass** bei Verwendung mehrerer Wärmetauscher (1, 2, 3) zur Verdampfung und Überhitzung der Edukte mindestens ein Wärmetauscher (1, 2, 3) im Gleichstrom und mindestens ein Wärmetauscher (1, 2, 3) im Gegenstrom arbeitet.

## Claims

1. A process for preparing methylamines by gas-phase reaction of methanol and ammonia as starting materials at a pressure in the range from 15 to 30 bar in the presence of a heterogeneous catalyst, which comprises vaporizing the starting materials in one or more heat exchangers (1, 2, 3), superheating them to form a feed gas stream and subsequently feeding this into a reactor (4), with the starting materials either being mixed in the feed stream to one of the heat exchangers (1, 2, 3) or at any other position on the heat exchanger (1, 2, 3), and taking off a product gas stream comprising monomethylamine, dimethylamine and trimethylamine and also reaction by-products from the reactor (4), wherein the reactor inlet temperature of the starting materials is regulated to a temperature in the range from 360°C to 370°C by passing part or all of the feed gas stream or product gas stream through an adjustable valve (5) installed upstream or downstream of the reactor in order to vary the pressure.

2. The process according to claim 1, wherein the valve (5) can be adjusted steplessly.

3. The process according to claim 1 or 2, wherein the product gas stream is used for vaporizing and superheating the starting materials, resulting in partial condensation of the product gas stream.

4. The process according to claim 1 or 2, wherein a substream of the product gas stream is used for vaporizing and superheating the starting materials.

5. The process according to any of claims 1 to 4, wherein the reaction by-products formed in the reaction are separated off from the product gas stream and fed back into the reactor (4).

6. The process according to claim 5, wherein the ammonia and the reaction by-products fed back into the reactor (4) are preheated before the methanol is added.

7. The process according to one or more of claims 1 to 6, wherein steam is added to the product gas stream to preheat and superheat the starting materials.

8. The process according to one or more of claims 1 to 7, wherein the reactor (4) is operated adiabatically.

9. The process according to one or more of claims 1 to 7, wherein heat of reaction is removed by cooling the reactor (4).

10. The process according to one or more of claims 1 to 9, wherein, when a plurality of heat exchangers (1, 2, 3) is used for vaporization and superheating of the starting materials, a droplet precipitator (6, 7) for separating condensate from the product gas stream is installed downstream of each heat exchanger (1, 2, 3).

11. The process according to one or more of claims 1 to 10, wherein the pressure at the inlet of the valve is from 0 to 5 bar higher than at the outlet of the valve.

12. The process according to one or more of claims 1 to 11, wherein the starting materials are heated up electrically to start the reaction.

13. The process according to one or more of claims 1 to 12, wherein vaporization and susperheating of the starting materials is carried out in countercurrent.

14. The process according to one or more of claims 1 to 12, wherein vaporization and superheating of the starting materials is carried out in cocurrent.

15. The process according to one or more of claims 1 to 12, wherein, when a plurality of heat exchangers (1, 2, 3) is used for vaporization and superheating of the starting materials, at least one heat exchanger (1, 2, 3) operates in cocurrent and at least one heat exchanger (1, 2, 3) operates in countercurrent.

## Revendications

1. Procédé de fabrication de méthylamines par réaction en phase gazeuse de méthanol et d'ammoniac en tant que réactifs en présence d'un catalyseur hétérogène à une pression dans la plage allant de 15 à 30 bar, selon lequel les réactifs sont vaporisés dans un ou plusieurs échangeurs de chaleur (1, 2, 3), surchauffés en un courant gazeux de réactifs, puis introduits dans un réacteur (4), les réactifs étant mélangés dans l'alimentation d'un des échangeurs de chaleur (1, 2, 3) ou à une autre position quelconque de l'échangeur de chaleur (1, 2, 3), un courant gazeux de produits contenant de la monométhylamine, de la diméthylamine et de la triméthylamine, ainsi que des produits de réaction secondaires, étant soutiré du réacteur (4), **caractérisé en ce que** pour le réglage de la température d'entrée dans le réacteur des réactifs à une température dans la plage allant de 360 °C à 370 °C, le courant gazeux de réactifs ou le courant gazeux de produits est passé en partie ou en totalité dans une valve ajustable (5), qui est montée avant ou après le réacteur (4), pour varier la pression.

2. Procédé selon la revendication 1, **caractérisé en ce que** la valve (5) est ajustable en continu.

3. Procédé selon la revendication 1 ou 2, **caractérisé en ce que** le courant gazeux de produits est utilisé pour la vaporisation et le surchauffage des réactifs, le courant gazeux de produits étant ainsi partiellement condensé.

4. Procédé selon la revendication 1 ou 2, **caractérisé en ce qu'**un courant partiel du courant gazeux de produits est utilisé pour la vaporisation et le surchauffage des réactifs.

5. Procédé selon l'une quelconque des revendications 1 à 4, **caractérisé en ce que** les produits de réaction secondaires formés lors de la réaction sont séparés du courant gazeux de produits et réintroduits dans le réacteur (4).

6. Procédé selon la revendication 5, **caractérisé en ce que** l'ammoniac et les produits de réaction secondaires réintroduits dans le réacteur (4) sont préchauffés avant l'ajout du méthanol.

7. Procédé selon une ou plusieurs des revendications 1 à 6, **caractérisé en ce que** de la vapeur d'eau est ajoutée au courant gazeux de produits pour le préchauffage et le surchauffage des réactifs.

8. Procédé selon une ou plusieurs des revendications 1 à 7, **caractérisé en ce que** le réacteur (4) est exploité de manière adiabatique.

9. Procédé selon une ou plusieurs des revendications 1 à 7, **caractérisé en ce que** la chaleur de réaction est dissipée par refroidissement du réacteur (4).

10. Procédé selon une ou plusieurs des revendications 1 à 9, **caractérisé en ce que** lors de l'utilisation de plusieurs échangeurs de chaleur (1, 2, 3) pour la vaporisation et le surchauffage des réactifs, un séparateur de gouttes (6, 7) est utilisé après chaque échangeur de chaleur (1, 2, 3) pour la séparation du condensat du courant gazeux de produits.

11. Procédé selon une ou plusieurs des revendications 1 à 10, **caractérisé en ce que** la pression à l'entrée de la valve est supérieure de 0 à 5 bar à la pression à la sortie de la valve.

12. Procédé selon une ou plusieurs des revendications 1 à 11, **caractérisé en ce que** les réactifs sont chauffés électriquement pour le démarrage de la réaction.

13. Procédé selon une ou plusieurs des revendications 1 à 12, **caractérisé en ce que** la vaporisation et le surchauffage des réactifs a lieu à contre-courant.

14. Procédé selon une ou plusieurs des revendications 1 à 12, **caractérisé en ce que** la vaporisation et le surchauffage des réactifs a lieu à co-courant.

15. Procédé selon une ou plusieurs des revendications 1 à 12, **caractérisé en ce que** lors de l'utilisation de plusieurs échangeurs de chaleur (1, 2, 3) pour la vaporisation et le surchauffage des réactifs, au moins un échangeur de chaleur (1, 2, 3) fonctionne à co-courant et au moins un échangeur de chaleur (1, 2, 3) à contre-courant.
